# EUROPEAN PATENT SPECIFICATION

(11) **EP 1 018 338 B1**
(45) Date of publication and mention of the grant of the patent: **27.11.2002**
(21) Application number: 98936435.1
(22) Date of filing: 05.08.1998
(51) Int. Cl.: A61K 31/20, A61P 43/00

(54) **USE OF A RETINOID TYPE COMPOUND TO INCREASE IN VIVO THE DECOUPLING ACTIVITY OF THE PROTEIN UCP2**
VERWENDUNG EINES RETINOIDIDS ZUR IN VIVO ERHÖHUNG DER ENTKOPPLUNG DES UCP2 PROTEINS
UTILISATION D'UN COMPOSE DE TYPE RETINOIDE POUR AUGMENTER IN VIVO L'ACTIVITE DE DESACCOUPLEMENT DE LA PROTEINE UCP2

(30) Priority: 05.08.1997 FR 9710009
(43) Date of publication of application: 12.07.2000
(73) Proprietor: CONSEJO SUPERIOR DE INVESTIGACIONES CIENTIFICAS, 28006 Madrid (ES); CENTRE NATIONAL DE LA RECHERCHE SCIENTIFIQUE, 75016 Paris Cédex (FR)
(72) Inventor: BOUILLAUD, Frédéric, C.N.R.S., 92190 Meudon (FR); RICQUIER, Daniel, C.N.R.S., 92190 Meudon (FR); RIAL ZUECO, Eduardo, C.S.I.C., 28006 Madrid (ES)
(74) Representative: Ungria Lopez, Javier
(86) International application number: ES9800225
(87) International publication number: WO99007358

(56) References cited:
- EP-A1- 0 579 915
- EP-A1- 0 698 392
- EP-A1- 0 749 752
- WO-A1-94/26277
- WO-A1-95/18533
- WO-A1-96/24344
- WO-A1-97/10819
- US-A- 5 219 888
- P. PUIGSERVER ET AL.: 'In vitro and in vivo induction of brown adipocyte uncoupling protein (thermogenin) by retinoic acid' BIOCHEM J., vol. 317, no. 3, 1996, pages 827 - 833
- R. ALVAREZ ET AL.: 'A novel regulatory pathway of brown fat thermogenesis' J. BIOL. CHEM., vol. 270, no. 10, 1995, pages 5666 - 5673
- R. RABELO ET AL.: 'A complex retinoic acid response element in the uncoupling protein gene defines a novel role for retinoids in thermogenesis' ENDOCRINOLOGY, vol. 137, no. 8, 1996, pages 3849 - 3496
- M.V. KUMAR ET AL.: 'Differential effects of retinoic acid on uncoupling protein and leptin gene expression' FASEB J., vol. 11, no. 3, April 1997, page A374
- G. WOLF.: 'A regulatory of thermogenesis in brown fat through retonoic acid' NUTRITION REV., vol. 53, no. 8, 1995, pages 230 - 231
- J.R. SILVA ET AL.: 'Regulation of the uncoupling protein gene expression' EUR. J. ENDOCRINOL., vol. 136, no. 3, 1997, pages 251 - 264
- C. FEURY ET AL.: 'Uncoupling protein-2 a novel gene linker to obesity and hyperinsulinemia' vol. 15, no. 3, March 1997, pages 269 - 272

## Description

This invention refers to new therapeutic applications of retinoid-type compounds, particularly of retinoic acid.

Retinoids and, particularly retinoic acid, are known as natural moleculae with important effects on cell differentiation. Retinoic acid (acid vitamin A) has found a very effective application in the treatment of severe forms of acne.

The new therapeutic applications considered for the retinoic acid point towards another field, i.e. that of disorders and diseases associated to the increase or decrease of the expression or the activity of uncoupling proteins.

Cellular respiration is a mechanism of oxidation of carbonated compounds which releases energy. It takes place in the mitochondria which are the organs responsible for cellular respiration. The so released energy is captured by the organism in diverse forms, particularly the synthesis of living matter, cell preservation etc. If recovery of the energy is defective, oxidation continues but a major proportion of this energy is immediately dissipated as heat. Rupture of the link between respiration and energy recovery at mitochondria level is named uncoupling. The coupling quality of mitochondria can be measured directly and in numerous cases a failure in this coupling can be observed, in respect of numerous organisms including plants and unicellular organisms, particularly in yeasts. Therefrom results an excess production of heat which is regulated by two types of mechanisms:
* a regulation of the genes encoded for certain proteins called uncoupling proteins or UCP proteins;
* or a direct activation of these very same proteins.

Various UCP proteins have already been identified as responsible for uncoupling respiration and thus for the dissipation of a portion of the energy as heat in mammals.

A first protein, UCP1, has been identified as being responsible of this mechanism in a particular organ, the brown adipose tissue, which is specialized in thermogenesis and active in certain animals and in human babies (Himms-Hagen-1. (1990) Brown adipose tissue thermogenesis, interdisciplinary studies, FASEB 1., 4, 2890-2898).

More recently other UCP proteins have been evidenced and particulary genes encoded for these proteins have been identified. These proteins have been named respectively UCP2, a protein expressed in almost all tissues (V. Fleury et al. (1997) Uncoupling protein-2: A novel gene linked to obesity and hyperinsuinaemia; Nature Genetics, 15, 269-272) and UCP3 expressed almost solely in the muscle (O. Boss et al. (1997) Uncoupling protein-3: A new member of mitchondrial carrier family with tissue-specific expression. FEBS Lett. 408, 39-42).

The activity of these UCP proteins i.e. their attitude to dissipate energy in the form of heat, achieves major therapeutic targets for the treatment of pathologies, disorders or diseases being associated to the regulation of energy consumption.

More particularly, the most interesting applications are the treatment of obesity which constitutes a major problem in the majority of industrialized countries, in such a degree that obesity is frequently associated to serious pathologies as some types of diabetes or even to hypertension.

In fact, recent reports appear to indicate that obesity is associated to an energetic unbalance. Calorimetric works in humans have also shown in obese persons, a diet-linked thermogenesis defect.

Therefore, since many years it has been aimed to mobilize the thermogenic mechanisms to fight accumulation of fat.

The inventors have now discovered that it was possible to act on uncoupling proteins by using retinoid-type compounds.

For this reason, it is the object of this invention to use a retinoid-type compound, particularly retinoic acid, for preparing a medicament being capable of *in vivo* modulating the uncoupling activity of UCP2 uncoupling proteins.

Modulation is to be understood as increasing the uncoupling activity.

Within the framework of this invention, "retinoid-type compound" is understood as compounds having an activity being similar to that of retinoic acid on the activity of UCP2 protein, taking into account particularly the negative tests which will be described hereinafter which appear to indicate the importance within the structure of the compound, of an acid function and of an preferably non-aromatic ring. These retinoid-type compounds particularly comprise compounds obtained starting from retinoic acid by deletion or substitution of certain elements of this acid. The expression "retinoid-type compounds" equally embraces metabolites of the compounds in question, and their precursors being capable of transforming in retinoid-type compounds at a cellular level.

The effect of this type of compounds particularly on the activity of UCP2 protein, has been shown on a yeast model. The test consists in constructing strains of recombinant *Saccharomyces cervisiae* yeasts being capable of expressing UCP2 protein, extracting UCP2 proteins from the mitochondria and measuring the activity of UCP2 protein in the presence or in the absence of retinoic acid. In this model, the uncoupling activity of the UCP2 proteins is quantitatively measured by means of the factor of stimulation of respiration.

This factor corresponds to the ratio between respiration and baseline respiration, measured in the absence of retinoic acid (when measurements are made at a constant pH but varying the concentration of retinoic acid) or at pH 6.8 (when measurements are made at a given concentration of retinoic acid, but varying the pH).

Respiration is determined by measurement of the quantity of consumed oxygen, expressed in nanomoles of oxygen per minute and milligram of mitochondrial proteins, the consumption of oxygen being directly linked to the quantity of substrate having been consumed by the mitochondria (in calories).

There is also a qualitative method for determining the uncoupling activity of an UCP protein. It is the measurement of the mitochondrial membrane potential, the reduction of which is an evidence of the decrease of the quality of the recovery of chemical energy at the level of the mitchondria. Activation of an uncoupling protein is reflected by de decrease of the membrane potential followed by an acceleration of the respiration.

The tests have been made in particular in respect of UCP2 protein and surprisingly it was observed that the retinoic acid activates the dissipation of energy by UCP2 acting directly on this protein.

Therefore, according to an advantageous embodiment, the invention refers to the use of a retinoid-type compound for preparing of a medicament being capable of increasing the uncoupling capacity of UCP2 protein.

Among the retinoid-type compounds useful according to the invention, preferably are cited retinoic acids and its derivatives and, still more preferably all trans and TTNPB retinoic acids.

More particularly, it is the object of the invention to use retinoic acid for preparing a medicament being capable of increasing the uncoupling activity of UCP2 protein.

Equally it has been verified that retinoic acid was capable of increasing the uncoupling activity of UCP1 protein.

Although this result may a *priori* be less interesting than that observed with UCP2 in terms of potential therapeutic applications due to the fact that UCP1 protein is scarcely expressed in humans, it does however allow to consider the conception of activators, being either common to various UCP proteins or specific to only one of them. In fact, the negative results referred to in the following examples, show that fatty acids, particularly palmitic acid, do not have an effect on the activation of UCP1 protein. However, retinoic acid acts on both UCP1 and UCP2.

This new activity of retinoid-type compounds allows to consider, and this constitutes one of the objects of the invention, a therapeutic application against pathologies associated with a reduction of the activity of UCP2 uncoupling proteins. In general, the term "pathologies" will be used to designate both physiological disorders and diseases linked to the perturbation of the uncoupling activity of uncoupling proteins.

More specifically, the retinoid-type compounds, particularly retinoic acid, may be used in the treatment of pathologies associated to the uncoupling activity of UCP2 protein.

As mentioned hereinbefore, obesity can be associated to an energetic unbalance which is the reason why the invention is directly applicable in an equally advantageous manner, in the treatment of obesity, as well as in the treatment of physiological disorders or of diseases linked to obesity. Anyway, in general the retinoid-type compounds defined according to the invention can be used in the treatment of all pathologies requiring a treatment aimed to increase energy consumption at a cellular level.

According to another embodiment, using a retinoid-type compound, particularly retinoic acid, may be also considered for esthetic purposes in humans or for modulating the energetic yield and the body composition of organisms of vegetal or animal agronomic interest. Thus, the use of a retinoid-type compound, particularly of retinoic acid, to control the energy balance in humans, animals or plants is also an object of the invention.

In the cited applications which point particularly towards the treatment of diverse pathologies, the preferred compounds are the same as those which have been cited as particularly advantageous for modulating the uncoupling activity of uncoupling proteins. These are particularly retinoic acids and their derivatives, particularly TTNPB and all-trans retinoic acids.

Finally, the invention refers more particularly to the use of retinoic acid for preparing a medicament for the treatment of obesity.

In the applications having been considered, the retinoid-type compounds may be dosed and formulated by convenient usual formulations for all commonly feasible administrations.

The invention will be illustrated by the following examples which evidence the effect of all-trans retinoic acid on the activities of UCP1 and UCP2 proteins in yeast mitochondria.

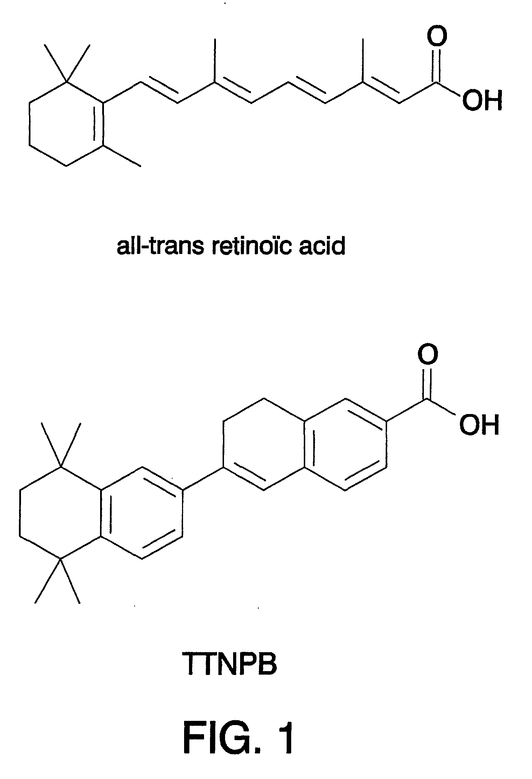

Figures 1 to 7 complete the illustration of the invention.

Figure 1 represents the structures of all-trans retinoic acid and of TTNPB retinoic acid which constitute examples of retinoid-type compounds according to the invention.

Figure 2 represents a curve of response of yeast mitochondria to retinoic acid. In the abscissa, there appears the molar ratio of retinoic acid / albumin (albumin concentration: 1 micromole within the medium); in the ordinate, there appears the factor of stimulation of the baseline respiration. Figure 2a was obtained at pH 6.8 and figure at pH 7.3.

Figure 3 is analogous to figure 2 but with palmitic acid constituting a negative comparative example as it appears that UCP2 does not respond to palmitic acid. In these curves
● represents the response in the absence of UCP protein (control);
□ represents the response in the presence of UCP1 protein;
▲ represents the response in the presence of UCP2 protein.

Figure 4 represents the same type of curves but in response to different pH, both at a molar ratio retinoic acid/albumin of 3:1 (fig. 4a) and at a molar ratio retinoic acid/albumin of 4:1 (fig. 4b), in the absence (·) or in the presence of UCP2 protein.

Figure 5 is analogous to figure 4 but it corresponds to a comparative with palmitic acid (figure 4a: molar ratio acid:albumin of 3:1; figure 4b: molar ratio palmitic acid:albumin of 4:1).

Figure 6 evidences the variation in the membrane potentials and respiration of mitochondria in yeasts (reflecting the uncoupling activity of UCP1 protein) under the effect of the retinoic acid. The upper curve reflects oxygen consumption (expressed in nanomoles of oxygen per minute per milligram of mitochondrial proteins) and the lower curve the membrane potentials. Figure 6a corresponds to measurements made in yeast mitochondria lacking UCP proteins and figure 6b corresponds to measurements made in yeast mitochondria wherein UCP1 protein is expressed.
- N:: designates the addition of NADH (respitation substrate)
- R:: designates the addition of retinoic acid (respectively at a molar ratio retinoic acid:albumin of 2:1 and 4:1)
- G:: designates the addition of 1 millimolar guanosine diphosphate (an inhibitor of UCP1 protein).

The time scale is 4 minutes and the basis of oxygen consumption is 100nmol oxygen.

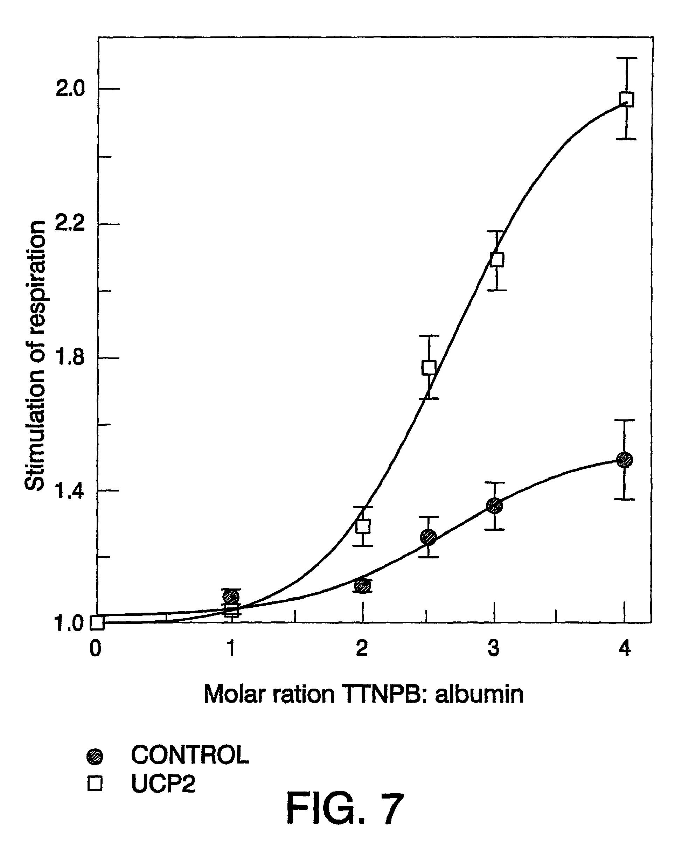

Figure 7 shows the relationship between TTNPB/albumin molar ratios (shown in the abscissa) and the factor of stimulation of respiration (shown in the ordinate).

The following examples illustrate on the one hand the uncoupling activity of UCP2 protein in the presence of all-trans retinoic acid, and the absence of such an increase in the presence of palmitic acid in mitochondria of *Saccharomyces cervisiae*. On the other hand, they evidence the increase of the uncoupling activity of UCP1 protein in the presence of retinoic acid and in the presence of palmitic acid in mitochondria of *Saccharomyces cervisiae.*

### Example 1: Construction of a model for the determination of the activity of UCP2 protein in yeasts

### 1. Materials and methods

In general, the restriction and modification enzymes are those commercialized by APPLIGENE or BIOLABS. Taq polymerase for amplification reactions by chain reaction is obtained from the company Perkin-Elmer. Also VENT DNA polymerases commercialized by BIOLABS can be used. The essential components of the culture media originate from LABORATORIOS DIFCO, cytohelicase comes from the company IBF BIOTECHNICS. The further reactants used are those of the highest possible purity.

### 2. Construction of strains of recombinant Saccharomyces cervisiae, capable of expressing UCP2 protein

The details and methods employed for constructing the strains and for selecting the recombinant one expressing UCP2 proteins are, by analogy, those described in the publication of Arechafa et al. (Biochem. J. (1993) 296, 693-700) which describes the expression of UCP1 protein in *Saccharomyces cervisiae* and the contents of which is incorporated herein by reference. Obtaining the control plasmid which does not produce any UCP2 protein is also described in this publication.

The cDNA of UCP2 proteins of mice (C. Fleury et al. (1997) Uncoupling protein-2: A novel gene linked to obesity and hyperinsulinaemia, Nature Genetics, 15, 269-272) lacking the untranslated regions 5' and 3'is obtained by enzymatic amplification with the following oligonucleotides:

5' primer: OL266 (aatcGAATTC atg ggt ttc aag gcc)

3' primer: OL267 (ggagGAGC TCa gaa agg tgc ctc ccg a).

The restriction sites EcoRI and Sacl are indicated in capital letters whereas the free spaces point out the reading planes.

The amplification product is the introduced into vector pYeDP 1/8-10 (Cullin C. et Pompon D., (1986) Gene 65, 203-217).

The resulting DNA product is repaired with a Kleenow enzyme, thereafter cut with EcoRl and Sacl. This forces clonation in the pYeDP-1/8-10 vector. In each case, the insertion of cDNA encoding for UCP2 protein in the pYeDP vector has been sequenced so as to verify that the encoding sequence had the expected properties. The UCP2-expressing pYeDP-YCP vector is introduced in a diploid W303 strain of *Saccharomyces cervisiae* yeast (a/β; ade2-10; his3-11,15; leu2-3,112; ura3-1; can1-100; trp-) by electroporation and the transformants are selected for their uracyl autotrophy.

### 3. Preparation of mitochondria

A process derived from that described by Guérin et al. (Guérin B., Labbe P. et Somlo, M. (1979) Methods Enzymol; 55, 149-159) is used. 36 hours before the extraction of the mitochondria a pre-culture in SP-medium (0.67% o nitrogenated yeast base, 0.1% KH2PO4, 0.12% (NH4)2S04, 0.1% glucose, 2% lactic acid, 0.1% casaminoacids, 20mg/l triptophane, 40mg/l adenine of pH 4.5), 10 or 12 hours before the extraction, a dilution in SP-medium (2% galactose, 0.67% nitrogenated yeast base, 0.1% casaaminoacids, 20mg/l triptophane and 40g/l adenine, at an optical density of 0.35-0.4).

Protoplasts are prepared by enzymatic digestion with cytohelicase and the mitochondria are isolated by differential centrifugation after homogenization of the protoplasts obtained by enzymatic digestion of the cell wall.

### 4. Measurement of the activity of UCP2

This activity may be measured qualitatively or quantitatively, with the help of the parameters:
- qualitatively:
   - The mitchondrial membrane potential; the increase of the activity of an uncoupling protein is reflected by a reduction of the membrane potential and a resulting increase in oxygen consumption. In turn, this increase corresponds to an increase in dissipation of energy.
- quantitatively:
   - mitochondrial respiration (oxygen consumption) which is linked directly to the quantity of substrate consumed by the mitochondria.

Only quantitative measurement of mitochondrial respiration has been made for UCP2. This measurement is made at 20ºC in an oxygen-electrode chamber (Clark electrode) commercialized by the company HANSATECH. The incubation medium contains 0.65M mannitol, 10nM Tris/Maleate, 0.5mM EGTA, 2mM MgC12, 10mMK2hpo4, 1mg/ml (16mM) albumin pH6.8).

Mitochondria concentration is 0.15 mg/ml. NADH (3mM) is used as substrate, since mitochondria of *Saccharomyces cervisiae* are capable of oxidizing NADH of external origin.

### Example 2: Measurement of the activity of UCP2 protein in the presence of all-trans retinoic acid.

The tests are performed varying either the pH or the retinoic acid concentration in the medium. The concentrations of free retinoic acid amount to some tens of nanomars (determined by fixing the molar ratio retinoic acid:albumin). The concentration of albumin is 0.1mg/ml (1.6mM) in the respiration buffer and the tested pH are respectively 6.8; 7.1; 7.3 or 7.5. The further components of the buffer are identical to those in example 1 and in the same concentrations.

### Example 3: Results

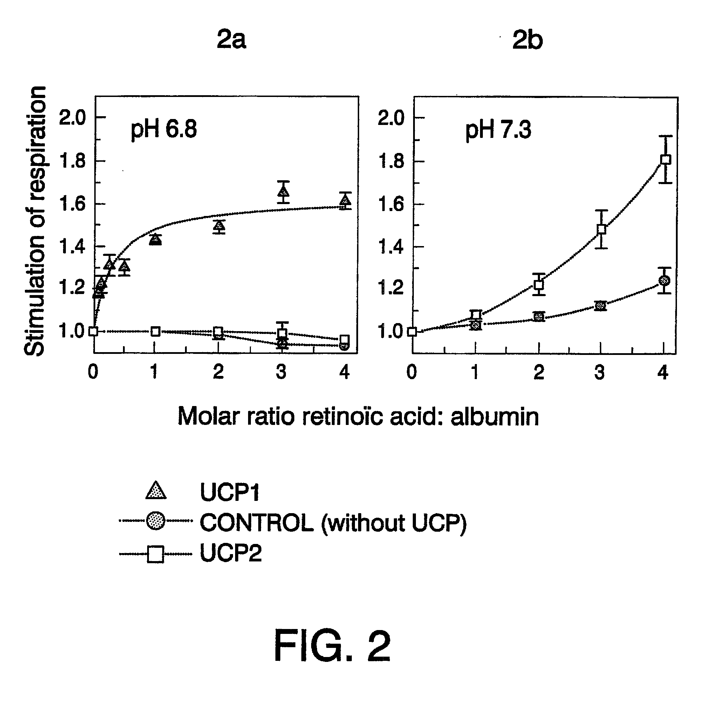

The results appear in figures 2 to 5 and indicate, by the value of the respiration stimulation factor, how much respiration is stimulated with reference to the respiration baseline value.

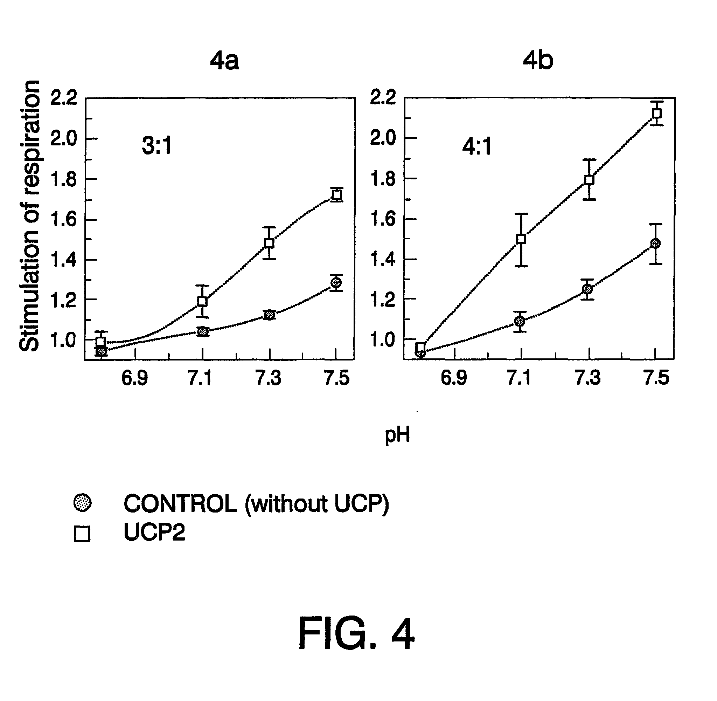

It is observed that activation of UCP2 in the presence of retinoic acid is strongly influenced by the pH (figures 2 and 4). There is no notable increase of the activity of UCP2 at pH 6.8. However, the increase is very sensitive at pH 7.3 which corresponds to the pH in the insides of the cells (figures 2a and 2b). The concentration of retinoic acid does not appear to play an essential role. Simply a slight increase of the activity of UCP2 protein is observed when the concentration of retinoic acid increases (figures 4a and 4b).

### Example 4:Comparative example with palmitic acid

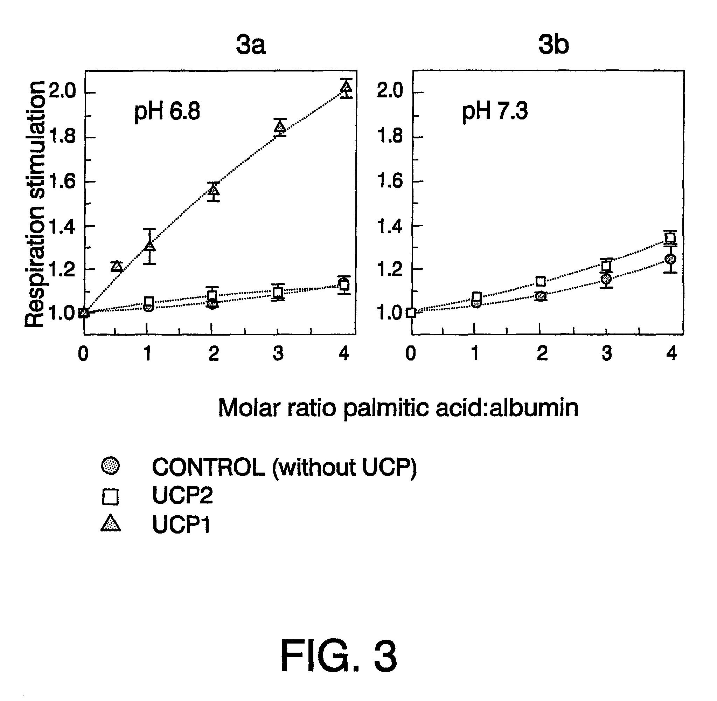

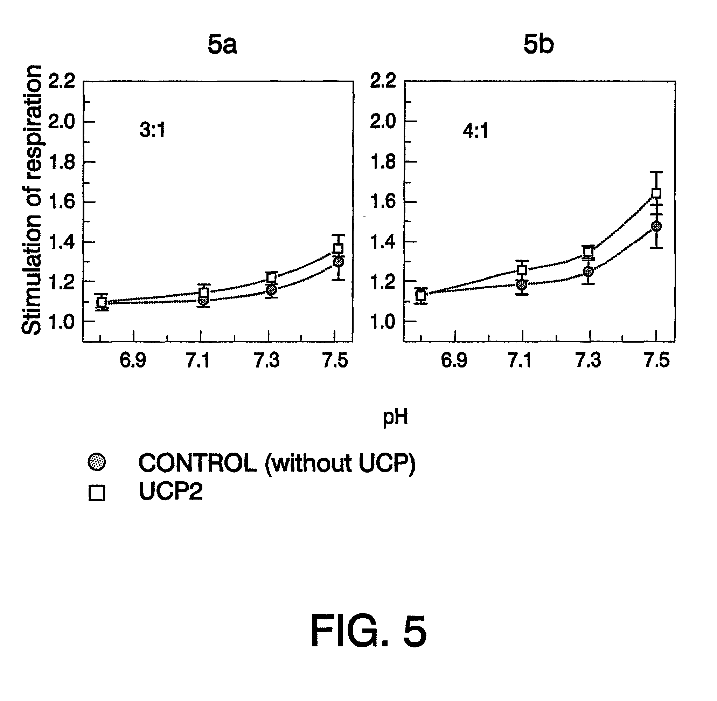

Figures 3b and 5 clearly indicate that palmitic acid does not have any effect on the activity of UCP2 protein. Simply a slight increase together with the pH and the increase of the concentration of the palmitic acid (figure 5) is observed.

### Example 5: Comparable tests were performed with other compounds. No activation of the activity of UCP2 protein in the presence of retinol, arachidonic acid, abscissic acid, indole acetic acid or salicylic acid could be verified.

### Example 6: Use of the model for the determination of the activity of UCP1 in the yeasts

The construction of the expression vectors, the recombinant *Saccharomyces cervisiae* strains, the preparation of the mitchondria and the measurement of the activity of the UCP1 protein are described in the publication of Arechaga et al. already cited in example 1. Particularly,the cDNA encoding for UCP1 protein between the internal site Sacl (110 nucleotides before the ATG codon) and the pstl site created during the cloning process (Bouillaud F. et al. Proc. Natl. Acad. Sci. U.S.A. 82, 445-448 et Bouillaud F. et al. (1986) J. Biol. Chem. 261, 1487-1490) is introduced in a pTZ 19 vector commercialized by PHARMACIA. The plasmid is linearized with Pstl, repaired with Kleenow enzyme and self-linked in the presence of an excess of EcoRl links (APPLIGENE). A plasmid ZlbRl is obtained wherein the cDNA can be removed with EcoRi. This insert is linked in the pSELECT vector (PROMAGA). The cDNA of the UCP1 protein which lacks the untranslated regions at 3' and 5' is obtained by enzymatic amplification with the following primers:

5' primer: OL5 (CGAGAAATTCATGGTTCGACAACTTC)

3' primer: OL6 (CAAGAATTCTATGTGGTGCAGTCCACTGT)

This process has allowed introduction of two EcoRl sites (italic) the closest possible to the ATG and TAG codons (underlined). After repairing with the Kleenow enzyme, a cut with EcoRl is made and the amplification product is introduced in the pYeDP-1/8-10 vector.

This final cloning step gives rise to two different plasmids:
- pYeDP-UCP+ in which it is expected that the sequence encoding for UCP2 gives a mRNA encoding for UCP1 protein, and
- pYeDP-UCP- in which the cDNA is in an opposite orientation, hence been expected to give rise to an antisense RNA.

The steps following of construction of the recombinant strains, of the preparation of the mitochondria and the measurement of the UVP1 activity are described in the publication of Arechaga et al.

### Example 7: Results on the activity of UCP1

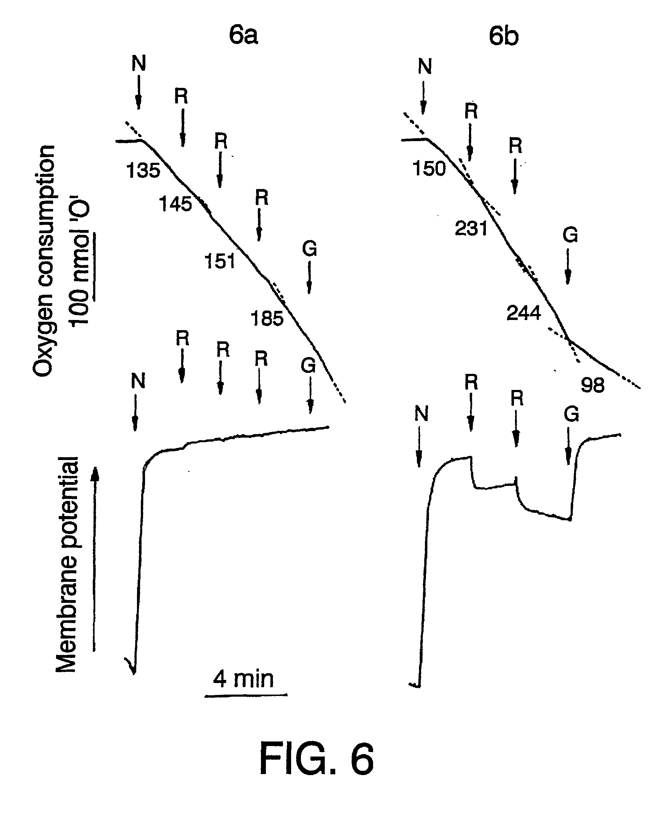

The results are reproduced partially in figures 2 and 3 as well as in figure 6.

In figure 2, it can be seen that the UCP1 protein responds to retinoic acid at pH=6.8 (figure 2a) which is not the case for the UCP2 protein.

Figure 3a evidences that UCP1 protein responds to palmitic acid which is not the case for UCP2 protein.

Finally, figure 6 shows that the addition of retinoic acid to the mitochondria of yeast induces a reduction of the membrane potential which indicates the the observed activity is an uncoupling phenomenon (figure 6b).

This effect is not observed in the mitochondria lacking UCP proteins (figure 6a).

## Claims

1. Use of a retinoid-type compound for the preparation of a medicament for increasing the uncoupling activity of UCP2 proteins.

2. Use according to claim 1, **characterized in that** said retinoid-type compound is selected from retinoic acids and derivatives thereof.

3. Use according to claim 2, wherein the retinoic acid is selected from TTNPB and all-trans retinoic acids.

4. Use of a retinoid-type compound according to any of claims 1 to 3, for preparing a medicament for treating pathologies associated to a reduction of the uncoupling activity of UCP2 proteins.

5. Use according to any of claims 1 to 3, wherein said medicament is for treating obesity.

6. Use according to any of claims 1 to 3, for the preparation of a medicament for increasing energy consumption at cellular level.

7. Use according to any of claims 1 to 3, for the preparation of a medicament for increasing mitochondrial respiratory activity.

8. Use according to claim 7, for the preparation of a medicament for increasing oxygen consumption by increasing mitochondrial respiratory activity.

## Patentansprüche

1. Verwendung einer retinoidartigen Verbindung zum Herstellen eines Arzneimittels zum Erhöhen der Entkopplungsaktivität von UCP2-Proteinen.

2. Verwendung nach Anspruch 1, **dadurch gekennzeichnet, dass** die retinoidartige Verbindung aus Retinsäuren und Derivaten davon ausgewählt wird.

3. Verwendung nach Anspruch 2, wobei die Retinsäure aus TTNPB und all-trans-Retinsäuren ausgewählt wird.

4. Verwendung einer retinoidartigen Verbindung nach einem der Ansprüche 1 bis 3 zum Herstellen eines Arzneimittels zum Behandeln von pathologischen Zuständen, die mit einer Verringerung der Entkopplungsaktivität von UCP2-Proteinen verbunden sind.

5. Verwendung nach einem der Ansprüche 1 bis 3, wobei das Arzneimittel zum Behandeln von Fettleibigkeit dient.

6. Verwendung nach einem der Ansprüche 1 bis 3 zum Herstellen eines Arzneimittels zum Erhöhen des Energieverbrauchs auf zellulärer Ebene.

7. Verwendung nach einem der Ansprüche 1 bis 3 zum Herstellen eines Arzneimittels zum Erhöhen der mitochondrialen Atmungsaktivität.

8. Verwendung nach Anspruch 7 zum Herstellen eines Arzneimittels zum Erhöhen des Sauerstoffverbrauchs durch Erhöhen der mitochondrialen Atmungsaktivität.

## Revendications

1. Utilisation d'un composé du type rétinoïde pour la préparation d'un médicament pour augmenter l'activité de découplage des protéines UCP2.

2. Utilisation selon la revendication 1, **caractérisée en ce que** ledit composé du type rétinoïde est choisi parmi les acides rétinoïques et leurs dérivés.

3. Utilisation selon la revendication 2, dans laquelle l'acide rétinoïque est choisi parmi le TTNPB et les acides rétinoïques tout-trans.

4. Utilisation d'un composé du type rétinoïde selon l'une quelconque des revendications 1 à 3, pour la préparation d'un médicament pour le traitement des pathologies associées à une réduction de l'activité de découplage des protéines UCP2.

5. Utilisation selon l'une quelconque des revendications 1 à 3, dans laquelle ledit médicament est destiné au traitement de l'obésité.

6. Utilisation selon l'une quelconque des revendications 1 à 3, pour la préparation d'un médicament pour augmenter la consommation d'énergie au niveau cellulaire.

7. Utilisation selon l'une quelconque des revendications 1 à 3, pour la préparation d'un médicament pour augmenter l'activité respiratoire mitochondriale.

8. Utilisation selon la revendication 7, pour la préparation d'un médicament pour augmenter la consommation mitochondriale par augmentation de l'activité respiratoire mitochondriale.
